# EUROPEAN PATENT APPLICATION

(11) **EP 2 165 695 A1**
(43) Date of publication of application: **24.03.2010**
(21) Application number: 08752724.8
(22) Date of filing: 14.05.2008
(51) Int. Cl.: A61K 8/37, A61K 8/06, A61K 8/33, A61K 8/39, A61K 8/891, A61Q 19/00, B01J 13/00

(54) **WATER-IN-OIL TYPE EMULSION COMPOSITION**

(30) Priority: 22.05.2007 JP 2007135231
(71) Applicant: Shiseido Company, Ltd., Chuo-ku Tokyo 104-8010 (JP)
(72) Inventor: WATANABE, Kei, Yokohama-shi Kanagawa 224-8558 (JP); SAKIGUCHI, Takayuki, Yokohama-shi Kanagawa 224-8558 (JP); TESHIGAWARA, Takashi, Yokohama-shi Kanagawa 224-8558 (JP)
(74) Representative: Lippert, Stachow & Partner
(86) International application number: PCT/JP2008/058853
(87) International publication number: WO 2008/143090

(57) **Abstract**

A water-in-oil type emulsion with a highly moisturizing effect on the skin has good emulsion stability and achieves a good feeling in use as a massage cream when a large amount of perfume is blended.

The water-in-oil type emulsion includes the following components:
(A) glyceryl monooleate,
(B) a surfactant having branched chains in an alkyl group,
(C) an aqueous component,
(D) an oil component, and
(E) a perfume component,
Conditions (1) and (2) are met by the emulsion. Condition (1): the ratio of the sum of glyceryl monooleate (component (A)) and the surfactant having branched chains in an alkyl group (component (B)) with respect to a total composition is from 5 to 10% by mass. Condition (2): the ratio of perfume component (component (E)) with respect to the total composition is 0.3 % by mass or more.

## Description

### RELATED APPLICATIONS

This application claims the priority of Japanese Patent Application No. 2007-135231 filed on May 22, 2007, which is incorporated herein by reference.

### FIELD OF THE INVENTION

The present invention relates to a water-in-oil type emulsion, and in particular, relates to an improved moisturizing effect on the skin, an improved emulsion stability when a large amount of perfume is blended, and an improved massaging ability when the emulsion is used as a massage cream.

### BACKGROUND OF THE INVENTION

An emulsion is broadly-divided into an oil-in-water (O/W) type and a water-in-oil (W/O) type. In addition to these types, there are multi-type emulsions such as oil/water/oil (O/W/O) type and water/oil/water (W/O/W) type. Conventionally, these emulsions have been utilized in a skin-care cream, a milky lotion, a massage cream, a makeup cleansing cream, and a hair-care cream in the cosmetic field and a transdermal cream in the pharmaceutical field.

A water-in-oil type emulsion, in which an oil phase constitutes the outer phase and a water phase constitutes the inner phase, is a suitable form as an external skin preparation because oil-soluble active ingredients such as an emollient oil, an oil-soluble drug, and a UV absorber can be efficiently spread on the skin. In this regard, a water-in-oil type emulsion is superior to an oil-in-water type emulsion.

It is generallythought that keeping a water-in-oil type emulsion stable is more difficult compared with an oil-in-water type emulsion (e.g., refer to Non-patent literature 1). In an oil-in-water type emulsion, entropic repulsion in polyoxyethylene chains of a polyoxyethylene-alkyl type surfactant, which is commonly used as a non-ion surfactant, or electrostatic repulsion in an ion surfactant can be utilized for stabilizing the emulsion. On the other hand, in a water-in-oil type emulsion, a lipophilic surfactant which is considered to be suitable for preparation tends to have low hydrophilicity (lipophobicity), and therefore a large ratio of the surfactant which should absorb to the interface dissolve in a monomolecular dispersed state in the oil phase to be consumed, resulting in a difficulty in an efficient stabilization in the oil-water interface. Hence, in an oil-in-water type emulsion, stabilization was often improved by blending polymers or a large amount of surfactant so that the oil which constituted the outer phase was gelated and droplets were immobilized. Thus, there has been a limitation in amounts and kinds of emulsifier, aqueous component, oil component, and other stabilizers as well as an inner water phase ratio.

When a perfume component is blended in a water-in-oil type emulsion, it is blended in an oil phase which constitutes the outer phase because a perfume is generally an oil component. A perfume is a mixture prepared by mixing natural perfumes which are highly-volatile oil components isolated from plants, synthetic perfumes as represented by terpenes, essential oils, and so on depending on a purpose. A perfume is classified as a polar oil component as it contains double bonds, alcohol,aldehyde, ketone, and so on in its molecular structure. Consequently, in an oil ingredient in which a large amount of perfume was blended, the lipophobic group (hydrophilic group) of a surfactant had an affinity for the oil ingredient, and the surfactant had a strong tendency to be dissolved in a monodispersed state, therefore there was the problem that stability of a water-in-oil type emulsion tended to deteriorate.

The skin-care effect of a massage cream utilizing a water-in-oil type emulsion is improved by applying the cream to the face or the body, and then massaging repeatedly for a certain period of time while spreading the cream with the fingers or the palms. However, in a conventional water-in-oil type massage cream, some of the components, such as water, vaporized during massaging, and a surfactant and oils, which were the remainingcomponents, had a large effect on feeling in use. Thus, it has been difficult to obtain a water-in-oil type massage cream which is excellent in both stability and feeling in use.

As described above, in a conventional water-in-oil type emulsion having an excellent skin-moisturizing effect, it was difficult to keep a good stability and have a good feeling in use as a massage cream when a large amount of perfume was blended.

On the other hand, a water-in-oil type emulsifiedcosmetic with a high stability has been developed by using a polyether-modified silicone as an emulsifier(e.g., refer to Patent literatures 1 and 2). In this technique, it is recommended to use a polyether-modified silicone having a high molecular weight (30000 or more). However, there has been a problem that a polyether-modified silicone generally behaved as a polymeric surfactant and gave a sticky feeling in use owing to entanglement between polymer chains.

Additionally, a water-in-oil type emulsion with a high internal water phase ratio, which was obtained by using partially-hydrophilized cross-linked polysiloxane, has been applied in cosmetics (e.g., refer to Non-patent literature 2). However, when an emulsion was prepared by using this technique, there has been a problem that the emulsion was easily destabilized by blending an oil component other than a cyclic silicone, such as a non-polar oil, a polar oil, or a linear silicone. Particularly, it was difficult to blend a large amount of perfume component, and there was a limitation in feeling in use.

Patent literature 1: Japanese Unexamined Patent Publication 2001-89356
Patent literature 2: Japanese Unexamined Patent Publication 2002-201355
Non-patent literature 1: Masahiko Abe, ed., Interface and Surfactant -from basic to application- (JAPAN Oil Chemists' Society, 2005)
Non-patent literature 2: Satsuki Kuribayashi, Oleo Science, Vol.1. No.3, 247-254 (2001)

### DISCLOSURE OF THE INVENTION

### PROBLEM TO BE SOLVED BY THE INVENTION

The present invention was made in view of the above-described problems of the conventional arts, and an object is to provide a water-in-oil type emulsion with a highly moisturizing effect on the skin which keeps a good emulsion stability and achieves a good feeling in use as a massage cream when a large amount of perfume is blended.

### MEANS TO SOLVE THE PROBLEM

The present inventors have diligently studied in view of the above-described problems of the conventional arts. As a result, the present inventors have found that a water-in-oil type emulsion containing (A) glyceryl monooleate, (B) a surfactant having branched chains in an alkyl group, (C) an aqueous component, (D) an oil component, and (E) a perfume component in a specific ratio achieves a highly moisturizing effect, particularly on the skin, a good emulsion stability even when a large amount of perfume is blended, and ease of massage when used as a massage cream, thus leading to completion of the present invention.

The present invention provides a water-in-oil type emulsion comprising following components (A), (B), (C), (D), and (E):
(A) glyceryl monooleate,
(B) a surfactant having branched chains in an alkyl group,
(C) an aqueous component,
(D) an oil component, and
(E) a perfume component,
wherein the water-in-oil type emulsion meets following Conditions (1) and (2):
Condition (1) is where the ratio of the sum of glyceryl monooleate as component (A) and a surfactant having branched chains in an alkyl group as component (B) with respect to the total composition is 5 % by mass or more to less than 10 % by mass, and
Condition (2) is where the ratio of the perfume component as component (E) with respect to the total composition is 0.3 % by mass or more.

In addition, it is preferable that the surfactant having iso-branched chains as component (B) in the water-in-oil type emulsion is polyoxyethylene isostearate, polyoxyethylene isostearyl ether, or polyoxyethylene glyceryl monoisostearate. In addition, it is preferable that the surfactant having iso-branched chains as component (B) has an average of 5 to 15 mol of polyoxyethylene portion in its molecular structure. Alternatively, it is preferable that the surfactant having iso-branched chains as component (B) in the water-in-oil type emulsion is tetramethyl trihydroxy hexadecane (trade name: Phytantriol).

In the water-in-oil type emulsion, it is preferable that the mass ratio of the total mass of components (A) and (B) with respect to the mass of component (E) is 10:1 to 10:5. The water-in-oil type emulsion comprises a cyclic silicone oil as one of the oil components (D). In addition, in the water-in-oil type emulsion, the total amount of glyceryl dioleate and glyceryl trioleate, which are contained as impurities of component (A), is less than 10 % by mass with respect to component (A).

A massage cream according to the present invention consists of the water-in-oil type emulsion.

### EFFECT OF THE INVENTION

A water-in-oil type emulsion according to the present invention comprises (A) glyceryl monooleate, (B) a surfactant having branched chains in an alkyl group, (C) an aqueous component, (D) an oil component, and (E) a perfume component in a specific ratio, and it achieves a highly moisturizing effect, particularly on the skin, a good emulsion stability even when a large amount of perfume is blended, and a good feeling in use when used as a massage cream.

### BEST MODE FOR CARRYING OUT THE INVENTION

A water-in-oil type emulsion according to the present invention comprises (A) glyceryl monooleate, (B) a surfactant having branched chains in an alkyl group, (C) an aqueous component, (D) an oil component, and (E) a perfume component, and it meets both conditions: 1) the ratio of the sum of glyceryl monooleate as component (A) and a surfactant having branched chains in an alkyl group as component (B) with respect to the total composition is 5 % by mass or more to less than 10 % by mass, and (2) the ratio of a perfume component as component (E) with respect to a total composition is 0.3 % by mass or more. In the following, each component is described in detail.

Glyceryl monooleate as component (A) can be obtained by various known synthesis methods. According to a common synthesis method, glyceryl monooleate is formed as a mixture of glyceryl monooleate, glyceryl dioleate, and glyceryl trioleate. It is desirable that glyceryl monooleate has a high degree of purity as component (A) of the water-in-oil type emulsion according to the present invention. A moleculardistillation method is generally used as a method for purifying glyceryl monooleate, however, the present invention is not limited thereto.

It is preferable that the total amount of glyceryl dioleate and glyceryl trioleate, which are present as impurities of component (A), is less than 25 % by mass with respect to component (A), and more preferably less than 10 % by mass. While glyceryl monooleate functions as a surfactant (i.e. an emulsifier), glyceryl dioleate and glyceryl trioleate behave as oils. Thus, when purity of glyceryl monooleate is low, emulsion stability tends to decreasein the same manner as when a polar oil is blended. Purity of glyceryl monooleate can be measured with a common method such as gas chromatography (GC), gel permeation chromatography (GPC), and high-performanceliquid chromatography (HPLC).

A surfactant having iso-branched chains as component (B) is preferably selected from polyoxyethylene isostearate, polyoxyethylene isostearyl ether, polyoxyethylene glyceryl isostearate, or tetramethyl trihydroxy hexadecane (trade name: Phytantriol). Tetramethyl trihydroxy hexadecane has four branched chains. Each of polyoxyethylene isostearate, polyoxyethylene isostearyl ether, and polyoxyethylene glyceryl isostearate has polyoxyethylene in its molecular structure. Crystallization at low temperature during preparation of an emulsion can be prevented by these structures. The mole number of the polyoxyethylene chain is preferably 5 to 15. When the mole number of the polyoxyethylene chain is less than 5, stability at low temperature is not sufficient. When it exceeds 15, hydrophilicity in the mixture of components (A) and (B) increases, which is unsuitable for preparing a water-in-oil type emulsion.

For a surfactant having iso-branched chains in an alkyl group as component (B), one or more surfactants generally used in cosmetics can be selected so far as stability does not deteriorate. The mass ratio between components (A) and (B) is preferably 1:1 to 4:1. By blending a surfactant having iso-branched chains in an alkyl group as component (B) into an emulsion of the present invention, the tendency of glyceryl monooleate as component (A) to be crystallized easily at low temperature is reduced and improved. When a large amount of component (B) is blended to decrease the mixing ratio between components (A) and (B) below 1:1, emulsion stability at high temperature becomes insufficient. In contrast, when a large amount of component (A) is blended to increase the mixing ratio between components (A) and (B) to above 4:1, crystallization at low temperature becomes a problem and stability becomes insufficient. Additionally, the greater the total amount of components (A) and (B), the more easily massage can be conducted and the better feeling in use becomes. It is preferable that the total amount of the pure component of monooleate which is contained in component (A) and component (B) is 10 to 5.0 % by mass with respect to the total mass, more preferably 7.0 to 5.0 % by mass. When it is less than 5.0 % by mass, ease of massage may not be sufficient. When it exceeds 10.0 % by mass, the emulsion may tend to have a sticky feeling.

For component (C), the aqueous component, which can be generally used for cosmetics, pharmaceuticals, and so on, can be blended so far as it doesn't deteriorate emulsion stability. Examples of moisturizer include 1,3-butylene glycol, polyethylene glycol, propylene glycol, dipropylene glycol, hexylene glycol, glycerin, diglycerin, xylitol, maltitol, maltose, and D-mannite. Examples of water-soluble polymer include plant-based polymers such as gum arabic, carrageenan, pectine, agar, quince seed (marmelo), starch, and algae colloid (brown algae extract), microorganism-based polymers such as dextran and pullulan, animal-based polymers such as collagen, casein, and gelatine, starch-based polymers such as carboxymethyl starch and methylhydroxypropyl starch, alginic acid-based polymers such as sodium alginate, vinyl-based polymers such as carboxy vinyl polymer (e.g., CARBOPOL®), polyoxyethylene-based polymers, polyoxyethylene/polyoxypropylene copolymer-based polymers, acryl-based polymers such as sodium polyacrylate and polyacrylamide, inorganic-based water-soluble polymers such as bentonite, magnesium aluminium silicate, and laponite.

Examples of UV absorber include benzoic acid UV absorbers such as p-aminobenzoicacid, anthranilic acid UV absorbers such as methyl anthranilate, salicylic acid UV absorbers such as octyl salicylate and phenyl salicylate, cinnamicacid UV absorbers such as isopropyl p-methoxycinnamate,octyl **p-methoxycinnamate,** and glyceryl mono-2-ethylhexanoate di-p-methoxycinnamate, benzophenone UV absorbers such as 2,4-dihydroxybenzophenone, 2-hydroxy-4-methoxybenzophenone, and 2-hydroxy-4-methoxybenzophenone-5-sulfonic acid, urocanic acid, 2-(2'-hydroxy-5'-methylphenyl)benzotriazole, and 4-tert-butyl-4'-methoxybenzoylmethane.

Examples of sequestering agent include sodium edetate, sodium metaphosphate, and phosphoric acid.
Examples of antioxidant include ascorbic acid, alpha-tocopherol, dibutylhydroxytoluene, and butylhydroxyanisole.

Examples of drugs include vitamins such as vitamin A oil, retinol, retinyl palmitate, inositol, pyridoxine hydrochloride, benzyl nicotinate, nicotinamide, dl-alpha-tocopherol nicotinate, magnesium ascorbyl phosphate, ascorbic acid 2-glucoside, vitamin D2 (ergocalciferol), L-ascorbic acid dl-alpha-tocopherol phosphoric acid diester potassium salt, dl-alpha-tocopherol, dl-alpha-tocopheryl acetate, pantothenic acid, and biotin, anti-inflammatory agents such as allantoin and azulene, whitening agents such as arbutin, astringent agents such as zinc oxide and tannic acid, sulfur, lysozyme chloride, pyridoxine hydrochloride, and gamma-orizanol.
The above-mentioned drugs can be used in a free state, a form of acid or basic salt if one can become salts, or a form of ester if one has a carbonic acid group.

For component (D), an oil component, which can be generally used for cosmetics, pharmaceuticals, and so on, can be blended so far as it doesn't deteriorate emulsion stability. The oil component is preferably a silicone oil, more preferably a cyclic silicone oil. Examples of silicone oil include liner silicone oils as represented by dimethylpolysiloxane, methylphenyl polysiloxane, and methylhydrogen polysiloxane, and cyclic silicone oils as represented by octamethylcyclotetrasiloxane, decamethylcyclopentasiloxane, and dodecamethylcyclohexasiloxane.
Examples of polar oil include ester oils as represented by cetyl octanoate, hexyl laurate, isopropyl myristate, octyl palmitate, isocetyl stearate, isopropyl isostearate, octyl isopalmitate, isodecyl oleate, 2-ethylhexyl succinate, and diethyl sebacate.
Examples of non-polar oil include hydrocarbon oils as represented by liquid paraffin, squalane, squalene, paraffin, isoparaffin, and ceresin.

For a perfume component as component (E), natural perfume and synthetic perfume can be used. Examples of natural perfume include plant-based perfumes isolated from flowers, leaves, wood, or pericarp, and animal-based perfumes such as musk and civet. Examples of synthetic perfume include hydrocarbons such as monoterpene, alcoholssuch as aliphatic alcohol and aromatic alcohol, aldehydes such as terpene aldehyde and aromatic aldehyde, ketones such as alicyclicketone, esters such as terpene esters, lactones, phenols, oxides, nitrogen-containing compounds, and acetals. These perfumes can be used alone, however, they are generally used in combination depending on a purpose.
The present invention provides a water-in-oil type emulsion wherein the mass ratio of the total mass of components (A) and (B) with respect to the mass of component (E) is 10:1 to 10:5. When a perfume component as component (E) is blended to decrease the ratio below 10:5, a sticky feeling occurs and massaging ability is deteriorated. When a perfume component as component (E) is blended to increase the ratio above 10:1, scent emanation of perfume becomes insufficient, which is not preferred.

In addition, the water-in-oil type emulsion of the present invention can be widely applied for cosmetics, pharmaceuticals, and quasi-drugs which are commonly applied to the skin. Examples of application include a whitening essence, a milky lotion, a cream, a pack, a foundation, a mascara, a makeup remover, a hair rinse, and a dermatological ointment. The water-in-oil type emulsion of the present invention can achieve a good feeling in use, especially during massaging when it is used as a massage cream.

EXAMPLE 1. The present invention is further described in the following Examples, however, the invention is not limited by these examples. Unless otherwise specified, a blending amount of a component is represented as mass % with respect to a system in which component is blended.

Preparation method of a water-in-oil type emulsion. Glyceryl monooleate as component (A), a surfactant having branched chains in an alkyl group as component (B), an oil component as component (D), a perfume component as component (E), and the other oil-soluble components are mixed and heated to about 40°C to be dissolved.An aqueous component as component (C) and the other water-soluble components are mixed and dissolved. The water-soluble component part is gradually added to the oil-soluble component part while the oil-soluble component part is stirred relatively strongly.

Evaluation method of a water-in-oil type emulsion. For the compositions showed in the following Tables 1 to 5, emulsion stability, moisturizing effect on the skin, feeling in use, and phase equilibrium were evaluated according to the evaluation criteria mentioned below.

### 1. Emulsion stability under high temperature

After each composition was preserved at 40 °C for one month, the emulsion stability was visually evaluated.
O : Separation of water and/or oil is not observed at all.
△ : Separation of water and/or oil can be observed slightly.
X : Separation of water and/or oil can be observed apparently.

### 2. Emulsion stability under low temperature

After each composition was preserved at 0 °C for one month, the emulsion stability was evaluated with an observation by optical microscope.
O : Crystal precipitation is not observed at all.
△ : Crystal precipitation can be observed slightly.
X : Crystal precipitation can be observed apparently.

### 3. Ease of massaging

10 professional panelists applied each water-in-oil type emulsion to their abdomen and judged ease of massaging.
⊚ : More than 9 panelists answered "good".
O : 6 or more and less than 9 panelists answered "good".
X : Less than 6 panelists answered "good".
4. Scent emanation of perfume

10 professional panelists applied each water-in-oil type emulsion to their abdomen and judged scent emanation of perfume.
O○: More than 9 of 10 panelists evaluated that the scent emanation of perfume was sufficient.
△ : 6 or more and less than 9 panelists evaluated that the scent emanation of perfume was sufficient.
X : Less than 6 panelists evaluated that the scent emanation of perfume was sufficient.

**Table 1**

| Components | 1-1 | 1-2 | 1-3 | 1-4 |
|---|---|---|---|---|
| Ion-exchanged water | 52.5 | 52.5 | 52.5 | 52.5 |
| Sodium chloride | 2 | 2 | 2 | 2 |
| Glyceryl monooleate(90 %) | - | - | - | 4 |
| POE(5) glyceryl monoisostearate | - | - | - | 1 |
| Sucrose oleate | 5 | - | - | - |
| Polyether-modified silicone | - | 5 | - | - |
| Diglyceryl diisostearate | - | - | 5 | - |
| Decamethylcyclopentasiloxane | 37 | 37 | 37 | 37 |
| Liquid paraffin | 3 | 3 | 3 | 3 |
| Perfume * | 0.5 | 0.5 | 0.5 | 0.5 |
| | | | | |
| Emulsion type | W/O | W/O | W/O | W/O |
| Emulsion stability (at 40 °C) | △ | △ | △ | ○ |
| Emulsion stability (at 0 °C) | O | O | O | O |
| Ease of massage | X | X | X | ⊚ |
| Scent emanation of perdume | O | O | O | O |

| | | | | |
|---|---|---|---|---|
| * a mixture of limonene,linalool, and benzyl acetate in the ratio of 1:1:1 | | | | |

As is clear from the results showed in the above Table 1, a water-in-oil type emulsion which contains 0.5 % of perfume can be prepared by selecting a surfactant with low HLB (such as sucrose oleate, polyether-modified silicone, or diglyceryl diisostearate). However, each of these water-in-oil type emulsions cannot achieve a sufficient emulsion stability at 40°C or a satisfactory ease of massage (Test Examples 1-1 to 1-3).
On the other hand, in Test Example 1-4, in which glyceryl monooleate and POE(5) glyceryl monoisostearate are used in combination, the obtained water-in-oil type emulsion had good emulsion stability at 40°C and was excellent in the ease of massage and scent emanation of perfume.
Consequently, the present inventors investigated the stabilization mechanism of an emulsion containing perfume in which glyceryl monooleate and POE(5) glyceryl monoisostearate (and other surfactants) were used in combination.

**Table 2**

| Components | 2-1 | 2-2 | 2-3 | 2-4 | 2-5 | 2-6 | 2-7 | 2-8 |
|---|---|---|---|---|---|---|---|---|
| Ion-exchanged water | 51 | 51.5 | 52.5 | 53.5 | 53.7 | 53.7 | 53.9 | 54 |
| Sodium chloride | 1.0 | 1.0 | 1.0 | 1.0 | 1.0 | 1.0 | 1.0 | 1.0 |
| Glyceryl monooleate (90 %) | 4.0 | 4.0 | 4.0 | 4.0 | 4.0 | - | - | - |
| POE(5) glyceryl monoisostearate | 1.0 | 1.0 | 1.0 | 1.0 | 1.0 | - | - | - |
| Diglyceryl diisostearate | - | - | - | - | - | 5.0 | 5.0 | 5.0 |
| Cetyl isooctanoate | 20 | 20 | 20 | 20 | 20 | 20 | 20 | 20 |
| Decamethylcyclopentasiloxane | 20 | 20 | 20 | 20 | 20 | 20 | 20 | 20 |
| Perfume * | 3 | 2.5 | 1.5 | 0.5 | 0.3 | 0.3 | 0.1 | 0 |
| | | | | | | | | |
| Ratio of (A)+(B):(E) | 10:6 | 10:5 | 10:3 | 10:1 | 10:0.6 | 10:0.6 | 10:0.2 | 10:0 |
| Emulsion type | W/O | W/O | W/O | W/O | W/O | W/O | W/O | W/O |
| Emulsion stability (at 40 °C) | O | O | O | O | O | △ | O | O |
| Emulsion stability (at 0 °C) | O | O | O | O | O | O | O | O |
| Ease of massage | O | ⊚ | ⊚ | ⊚ | ⊚ | X | X | X |
| Scent emanation of perfume | O | O | O | O | △ | △ | X | X |

| | | | | | | | | |
|---|---|---|---|---|---|---|---|---|
| * a mixture of limonene,linalool, and benzyl acetate in the ratio of 1:1:1 1 | | | | | | | | |

As showed in the above Table 2, in Test Example 2-1, in which (A) + (B) : (E) was 10:6, the massaging ability was slightly deteriorated. In addition, in Test Example 2-5, in which the ratio of (A) + (B) : (E) was 10:0.6, the stability and the massaging ability were good, however, the scent emanation of perfume was deteriorated. In Test Examples 2-6 to 2-8, in which diglyceryl diisostearate was used as an emulsifier, the massaging ability and the scent emanation of perfume were not sufficient while the emulsion stability was improved by reducing the blending amount of perfume component.
Consequently, the present inventors further investigated a relationship between an amount of emulsifier in a water-in-oil type emulsion and emulsion stability or massaging ability.

**Table 3**

| Components | 3-1 | 3-2 | 3-3 | 3-4 | 3-5 | 3-6 | 3-7 | 3-8 |
|---|---|---|---|---|---|---|---|---|
| Ion-exchanged water | 60 | 57 | 56 | 51 | 46 | 58 | 53 | 48 |
| Sodium chloride | 2 | 2 | 2 | 2 | 2 | - | - | - |
| Glyceryl monooleate (90 %) | 0.8 | 3.2 | 4.0 | 8.0 | 12.0 | - | - | - |
| POE(5)glyceryl isostearate | 0.2 | 0.8 | 1.0 | 2.0 | 3.0 | - | - | - |
| Diglyceryl diisostearate | - | - | - | - | - | 5.0 | 10.0 | 15.0 |
| Perfume * | 1 | 1 | 1 | 1 | 1 | 1 | 1 | 1 |
| Decamethylcyclopentasiloxane | 22 | 22 | 22 | 22 | 22 | 22 | 22 | 22 |
| Liquid paraffin | 13 | 13 | 13 | 13 | 13 | 13 | 13 | 13 |
| Dimethylsiloxane | 1 | 1 | 1 | 1 | 1 | 1 | 1 | 1 |
| | | | | | | | | |
| Emulsion type | W/O | W/O | W/O | W/O | W/O | W/O | W/O | W/O |
| Emulsion stability (at 40 °C) | O | O | O | O | O | △ | O | O |
| Emulsion stability (at 0 °C) | O | O | O | O | O | O | O | O |
| Ease of massage | X | △ | O | O | O | X | X | X |
| Scent emanation of perfume | O | O | O | O | △ | O | O | △ |

| | | | | | | | | |
|---|---|---|---|---|---|---|---|---|
| * a mixture of limonene,linalool, and benzyl acetate in the ratio of 1:1:1 1 | | | | | | | | |

As showed in the above Table 3, the massaging ability tended to be deteriorated when the total blending amount of glyceryl monooleate as component (A) and POE (5) glyceryl isostearate as component (B) was less than 5 % (Test Examples 3-1 and 3-2). When the total blending amount of glyceryl monooleate as component (A) and POE (5) glyceryl isostearate as component (B) was 15 %, the scent emanation of perfume was deteriorated because the ratio between the total amount of components (A) and (B) and component (E) became outside the range of 10:5 to 10:1 (Test Example 3-5). In Test Examples 3-6 to 3-8, in which diglyceryl diisostearate was used, the massaging ability was not improved even when the blending amount was increased.
Consequently, the present inventors further investigated a relationship between a kind of surfactant used for preparing a water-in-oil type emulsion and emulsion stability or massaging ability.

**Table 4**

| Components | 4-1 | 4-2 | 4-3 | 4-4 | 4-5 | 4-6 | 4-7 |
|---|---|---|---|---|---|---|---|
| Ion-exchanged water | 51 | 51 | 51 | 51 | 51 | 51 | 51 |
| Potassium chloride | 2 | 2 | 2 | 2 | 2 | 2 | 2 |
| Glyceryl monooleate (90 %) | 5 | 5 | 5 | 5 | 5 | 5 | 5 |
| Tetramethyl trihydroxy hexadecane | 5 | - | - | - | - | - | - |
| POE(5) isostearate | - | 5 | - | - | - | - | - |
| POE(5) isostearyl ether | - | - | 5 | - | - | - | - |
| POE(5) glyceryl isostearate | - | - | - | 5 | - | - | - |
| POE(5) laurate | - | - | - | - | 5 | - | - |
| POE(5) stearate | - | - | - | - | - | 5 | - |
| Isostearyl glyceryl ether | - | - | - | - | - | - | 5 |
| Decamethylcyclopentasiloxane | 20 | 20 | 20 | 20 | 20 | 20 | 20 |
| Cetyl isooctanoate | 16 | 16 | 16 | 16 | 16 | 16 | 16 |
| Perfume * | 1 | 1 | 1 | 1 | 1 | 1 | 1 |
| | | | | | | | |
| Emulsion type | W/O | W/O | W/O | W/O | W/O | W/O | W/O |
| Emulsion stability (at 40 °C) | O | O | O | O | O | O | O |
| Emulsion stability (at 0 °C) | O | O | O | O | X | X | △ |
| Ease of massage | ⊚ | ⊚ | ⊚ | ⊚ | X | X | X |
| Scent emanation of perfume | O | O | O | O | O | O | O |

As showed in the above Table 4, emulsion stability was investigated in each composition in which glyceryl monooleate (90 %) was used as one kind of the surfactants and the other hydrophilic surfactant was varied among the compositions.
As a result of this investigation, it was found that, when tetramethyl trihydroxy hexadecane was used, the emulsion stability, the ease of massage, and the scent emanation of perfume were excellent (Test Example 4-1). Next, when a surfactant with an adequate alkyl chain length having polyoxyethylene structure, which was commonly used as an emulsifierin cosmetics, was selected, the resulting compositions had an excellent emulsion stability, such as Test Examples 4-2 to 4-4. However, even if a surfactant having similar polyoxyethylene structure and a similar alkyl chain length was used, crystallization under low temperature became a problem and the emulsion stability was deteriorated when an alkyl group of the surfactant was not branched (Test Examples 4-5 and 4-6). Even if a surfactant having a similar alkyl group with branched chain was used, the emulsion stability under low temperature was slightly deteriorated when the surfactant didn't have polyoxyethylene structure (Test Example 4-7). In each of the compositions using these surfactants, the evaluation for massaging ability was not sufficient.
From these results, it can be understood that, for a surfactant which is used in combination with glyceryl monooleate, tetramethyl trihydroxy hexadecane or a surfactant having polyoxyethylene structure and branched structure in its alkyl group is preferably used for preparing a water-in-oil type emulsion which is stable and has a good massaging ability even when a large amount of perfume is blended.

## Claims

1. A water-in-oil type emulsion comprising following components (A), (B), (C), (D), and (E):
(A) glyceryl monooleate,
(B) a surfactant having branched chains in an alkyl group,
(C) an aqueous component,
(D) an oil component, and
(E) a perfume component,
wherein the water-in-oil type emulsion meets following Conditions (1) and (2): Condition (1) a ratio of a sum of glyceryl monooleate as component (A) and a surfactant having branched chains in an alkyl group as component (B) with respect to a total composition is 5 % by mass or more to less than 10 % by mass, and Condition (2) a ratio of a perfume component as component (E) with respect to a total composition is 0.3 % by mass or more.

2. The water-in-oil type emulsion according to Claim 1, wherein the surfactant having iso-branched chains as component (B) is polyoxyethylene isostearate.

3. The water-in-oil type emulsion according to Claim 1, wherein the surfactant having iso-branched chains as component (B) is polyoxyethylene isostearyl ether.

4. The water-in-oil type emulsion according to Claim 1, wherein the surfactant having iso-branched chains as component (B) is polyoxyethylene glyceryl monoisostearate.

5. The water-in-oil type emulsion according to any of Claims 1 to 4, wherein the surfactant having iso-branched chains as component (B) has an average of 5 to 15 mol of polyoxyethylene portion in its molecular structure.

6. The water-in-oil type emulsion according to Claim 1, wherein the surfactant having iso-branched chains as component (B) is tetramethyl trihydroxy hexadecane.

7. The water-in-oil type emulsion according to any of Claims 1 to 6, wherein a mass ratio of a total mass of components (A) and (B) with respect to a mass of component (E) is 10:1 to 10:5.

8. The water-in-oil type emulsion according to any of Claims 1 to 7, comprising a cyclic silicone oil as one of the oil components (D).

9. The water-in-oil type emulsion according to any of Claims 1 to 8, wherein a total amount of glyceryl dioleate and glyceryl trioleate, which are contained as impurities of component (A), is less than 10 % by mass with respect to component (A).

10. A massage cream, consisting of the composition according to any of Claims 1 to 9.
